# FASCICULE DE BREVET EUROPEEN

(11) **EP 0 859 591 B1**
(45) Date de publication et mention de la délivrance du brevet: **20.08.2003**
(21) Numéro de dépôt: 96933468.9
(22) Date de dépôt: 01.10.1996
(51) Int. Cl.: A61K 7/48

(54) **UTILISATION D'AU MOINS UN INHIBITEUR DE NO-SYNTHASE DANS LE TRAITEMENT DES PEAUX SENSIBLES**
VERWENDUNG VON WENIGSTENS EINEM NO-SYNTHASE HEMMER ZUR BEHANDLUNG EMPFINDLICHER HAUT
USE OF AT LEAST ONE NO SYNTHASE INHIBITOR FOR TREATING SENSITIVE SKIN

(30) Priorité: 26.10.1995 FR 9512653
(43) Date de publication de la demande: 26.08.1998
(73) Titulaire: L'OREAL, 75008 Paris (FR)
(72) Inventeur: BRETON, Lionel, F-78000 Versailles (FR); DE LACHARRIERE, Olivier, F-75015 Paris (FR)
(74) Mandataire: Tezier Herman, Béatrice
(86) Numéro de dépôt international: FR9601529
(87) Numéro de publication internationale: WO97015280

(56) Documents cités:
- EP-A- 0 096 521
- EP-A- 0 249 736
- EP-A- 0 413 528
- EP-A- 0 630 649
- WO-A-95/13805
- WO-A-95/34534
- US-A- 5 449 688

## Description

La présente invention concerne l'utilisation d'au moins un inhibiteur de NO-synthase dans une composition cosmétique à titre de principe actif pour traiter et/ou prévenir les peaux sensibles, ou pour la préparation d'une composition pharmaceutique, plus particulièrement dermatologique, destinée à traiter les peaux sensibles.

Dans le domaine des désordres cutanés, il est connu que certaines peaux sont plus sensibles que d'autres. Toutefois les symptômes des peaux sensibles étaient jusqu'à présent mal caractérisés et le problème de ces peaux était, de ce fait, mal défini ; personne ne connaissait exactement le processus mis en cause dans la sensibilité de la peau. Certains pensaient qu'une peau sensible était une peau qui réagissait aux produits cosmétiques, d'autres qu'il s'agissait d'une peau qui réagissait à plusieurs facteurs extérieurs, pas forcément liés aux produits cosmétiques. On assimilait également les peaux sensibles à des peaux allergiques.

Des tests ont été mis au point pour cerner les peaux sensibles, par exemple des tests à l'acide lactique et au DMSO qui sont connus pour être des substances ïrritantes : voir par exemple l'article de K. Lammintausta et al., Dermatoses, 1988, 36, pages 45-49; et l'article de T. Agner et J. Serup, Clinical and Experimental Dermatology, 1989, 14, pages 214-217.

Du fait de la méconnaissance des caractéristiques des peaux sensibles, il était jusqu'à présent très difficile voire impossible de les traiter. En fait, on les traitait indirectement, par exemple en limitant dans les compositions cosmétiques ou dermatologiques l'emploi de produits à caractère irritant tels que les tensioactifs, les conservateurs ou les parfums ainsi que l'emploi de certains actifs cosmétiques ou dermatologiques.

Après de nombreux tests cliniques, la demanderesse a pu déterminer les symptômes liés aux peaux sensibles. Ces symptômes sont en particulier des signes subjectifs, qui sont essentiellement des sensations dysesthésiques. On entend par sensations dysesthésiques des sensations plus ou moins douloureuses ressenties dans une zone cutanée comme les picotements, fourmillements, démangeaisons ou prurits, brûlures, échauffements, inconforts, tiraillements, etc.

La demanderesse a pu montrer en outre qu'une peau sensible n'était pas une peau allergique. En effet, une peau allergique est une peau qui réagit à un agent extérieur, un allergène, qui déclenche une réaction d'allergie. Il s'agit d'un processus immunologique qui ne se produit que lorsqu'un allergène est présent et qui ne touche que les sujets sensibilisés. La caractéristique essentielle de la peau sensible est selon la demanderesse, au contraire, un mécanisme de réponse à des facteurs extérieurs, qui peut concerner tout individu, même si les individus dits à peau sensible y réagissent plus vite que les autres. Ce mécanisme n'est pas immunologique, il est aspécifique.

La demanderesse a trouvé que les peaux sensibles pouvaient être scindées en deux grandes formes cliniques, les peaux irritables et/ou réactives, et les peaux intolérantes.

Une peau irritable et/ou réactive est une peau qui réagit par un prurit, c'est-à-dire par des démangeaisons ou par des picotements, à différents facteurs tels que l'environnement, les émotions, les aliments, le vent, les frottements, le rasoir, le savon, les tensioactifs, l'eau dure à forte concentration de calcaire, les variations de température ou la laine. En général, ces signes sont associés à une peau sèche avec ou sans dartres ou à une peau qui présente un érythème.

Une peau intolérante est une peau qui réagit par des sensations d'échauffement, de tiraillements, de fourmillements et/ou de rougeurs, à différents facteurs tels que l'environnement, les émotions, les aliments et certains produits cosmétiques. En général, ces signes sont associés à une peau hyperséborrhéique ou acnéique avec ou sans dartres et à un érythème.

Les cuirs chevelus "sensibles" ont une sémiologie clinique plus univoque : les sensations de prurit et/ou de picotements et/ou d'échauffements sont essentiellement déclenchés par des facteurs locaux tels que frottements, savon, tensioactifs, eau dure à forte concentration de calcaire, shampooings ou lotions. Ces sensations sont aussi parfois déclenchées par des facteurs tels que l'environnement, les émotions et/ou les aliments. Un érythème et une hyperséborrhée du cuir chevelu ainsi qu'un état pelliculaire sont fréquemment associés aux signes précédents.

Par ailleurs, dans certaines régions anatomiques comme les grands plis (régions inguinales, génitale, axillaires, poplitées, anale, sous-mammaires, plis du coude) et les pieds, la peau sensible se traduit par des sensations prurigineuses et/ou des sensations dysesthésiques (échauffement, picotements) liées en particulier à la sueur, aux frottements, à la laine, aux tensioactifs, à certaines préparations cosmétiques, à l'eau dure à forte concentration en calcaire et/ou aux variations de température.

Pour déterminer si une peau est sensible ou non, la demanderesse a également mis au point un test. En effet, après avoir effectué un grand nombre de tests dans le but de définir une peau sensible, elle a trouvé qu'il existait un lien entre les personnes à peau sensible et celles qui réagissaient à une application topique de capsaïcine.

Le test à la capsaïcine consiste à appliquer sur environ 4 cm² de peau 0,05 ml d'une crème comprenant 0,075 % de capsaïcine et à noter l'apparition de signes subjectifs provoqués par cette application, tels que picotements, brûlures et démangeaisons. Chez les sujets à peaux sensibles, ces signes apparaissent entre 3 et 20 minutes après l'application et sont suivis de l'apparition d'un érythème qui débute à la périphérie de la zone d'application.

Jusqu'à présent, la capsaïcine était utilisée comme médicament, en particulier pour traiter les douleurs du zona. La capsaïcine provoque un relargage des neuropeptides, et en particulier des tachykinines qui proviennent de terminaisons nerveuses de l'épiderme et du derme. La demanderesse a constaté que le schéma physiopathologique commun à tous les états des peaux sensibles était l'aptitude à libérer à partir des fibres nerveuses sensitives cutanées (fibre C), différents médiateurs biologiques tels que les tachykinines notamment la substance P, le peptide dérivé de la calcitonine (CGRP) et/ou le monoxyde d'azote (NO), ce dernier étant libéré sous la dépendance d'une NO-synthase constitutive. On sait en outre que la substance P libérée par les terminaisons sensitives épidermiques induit une dégranulation des cellules sanguines impliquées dans l'inflammation (mastocytes, monocytes, macrophages) avec libération de différents médiateurs comme l'histamine, la serotonine, les interleukines, l'héparine, le facteur de nécrose tumorale de type α (TNF-α). Cette cascade d'événements biochimiques aboutit à une réaction inflammatoire dans laquelle le monoxyde d'azote (libéré sous la dépendance d'une NO-synthase inductible) est également impliqué. Les manifestations dysesthésiques qui sont ainsi provoquées sont dites "neurogènes".

Personne n'avait établi un lien entre le monoxyde d'azote (NO) et la peau sensible. Les signes cliniques de la peau sensible sont essentiellement subjectifs : picotements, fourmillements, prurits, tiraillements, échauffements, et ils s'associent parfois à des érythèmes. Ces signes sont dus à des facteurs extérieurs aspécifiques. Les symptômes apparaissent essentiellement localisés au visage, au cou et au cuir chevelu, mais peuvent apparaître aussi sur tout le corps.

Ainsi, la demanderesse a découvert que l'une des caractéristiques essentielles des peaux sensibles est liée à la libération de monoxyde d'azote et donc que l'utilisation d'inhibiteur de l'enzyme liée à cette libération, la NO-synthase, peut permettre d'obtenir un effet préventif et/ou curatif des peaux sensibles.

Le terme NO-synthase recouvre en fait une famille d'enzymes qui de façon spécifique des tissus assurent la catalyse enzymatique de la L-arginine en citrulline, catalyse au cours de laquelle est produit un médiateur gazeux aux multiples fonctions, le monoxyde d'azote ou NO. Le monoxyde d'azote possède de par sa structure un électron supplémentaire le rendant extrêmement réactif chimiquement. Il est notoire que de tels composés sont nocifs et l'on cherche à limiter au mieux leur production. C'est ainsi que dans le cas du monoxyde d'azote les inhibiteurs de NO-synthase ont été largement étudiés.

Les NO-synthases existent sous deux formes, une forme constitutive, nomenclature regroupant la NO-synthase neuronale (ou NOS 1) et la NO-synthase endothéliale (ou NOS 3), et la forme inductible (ou NOS 2) (Medecine/Sciences, 1992, 8, pp. 843-845). Selon l'invention, on utilise des inhibiteurs de la forme constitutive ou de la forme inductible. Les tests pour identifier les inhibiteurs de NO-synthase constitutive ou inductible sont notamment décrits dans le brevet US 5132453.

Les inhibiteurs de NO-synthases sont donc choisis parmi les composés inhibant la synthèse et/ou accélérant le catabolisme de la NO-synthase, les composés neutralisant la NO-synthase ou les composés intervenant en modulant le signal transduit par la NO-synthase.

Ainsi, selon l'invention les inhibiteurs de NO-synthase sont des produits qui permettent *in situ* sur l'homme d'inhiber partiellement, voire totalement, la synthèse de monoxyde d'azote (NO).
Pour traiter les peaux sensibles, la demanderesse a donc envisagé d'utiliser des inhibiteurs de la NO-synthase. Elle a en effet constaté de manière surprenante que l'incorporation d'un inhibiteur de la NO-synthase dans une composition destinée à un usage topique permet d'éviter l'irritation et/ou les sensations dysesthésiques et/ou les prurits de la peau.

L'invention concerne donc plus particulièrement l'utilisation d'au moins un inhibiteur de NO-synthase pour la préparation d'une composition cosmétique ou pharmaceutique destinée au traitement des peaux irritables et/ou réactives et/ou intolérantes.

Ainsi, l'inhibiteur de la NO-synthase peut être choisi parmi des peptides, synthétiques ou naturels, éventuellement modifiés, des molécules chimiques, synthétiques ou naturelles, des acides nucléiques antisens, des ribozymes, des anticorps anti-NO-synthase.

Parmi ces inhibiteurs de la NO-synthase, on peut citer notamment
la N^{G}-monométhyl-L-arginine (NMMA), la N^{G}-nitro-L-arginine,
l'ester méthylé de la N^{G}-nitro-L-arginine,
le chlorure de diphénylèneiodonium, la 7-nitroindazole,
la N(5)-(1-iminoéthyl)-L-ornithine, la NG,NG-diméthyl-L-arginine,
la NG,NG-diméthyl-arginine,
le 2-(4-carboxyphényl)-4,4,5,5-tetraméthylimidazoline-1-oxy-3-oxyde,
l'aminoguanidine, la canavanine et l'ebselen.

Parmi les inhibiteurs de la NO-synthase, on utilise préférentiellement l'ester méthyle de la N^{G}-nitro-L-arginine ou la NG,NG-diméthyl-arginine.

Les inhibiteurs de la NO-synthase peuvent être utilisés seuls ou en mélange.

La présente invention a encore pour objet l'utilisation d'au moins un inhibiteur de NO-synthase dans une composition cosmétique ou pour la préparation d'une composition pharmaceutique, destinée à prévenir et/ou à lutter contre les irritations cutanées et/ou les dartres et/ou les érythèmes et/ou les sensations d'échauffement et/ou de dysesthésie et/ou les prurits de la peau et/ou les muqueuses.

Selon l'invention, l'inhibiteur de NO-synthase peut être utilisé en une quantité pondérale représentant de 10⁻⁶ % à 10 % du poids total de la composition et préférentiellement en une quantité représentant de 10⁻⁴ % à 1 % du poids total de la composition.

L'inhibiteur de NO-synthase peut être utilisé dans une composition qui doit être ingérée, injectée ou de préférence appliquée sur la peau (sur toute zone cutanée du corps), les cheveux, les ongles ou les muqueuses (buccale, jugale, gingivale, génitale, anale, conjonctive). Selon le mode d'administration, cette composition peut se présenter sous toutes les formes galéniques normalement utilisées.

Pour une application topique sur la peau, la composition peut avoir la forme notamment de solution aqueuse ou suspension huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique. Ces compositions sont préparées selon les méthodes usuelles.

Elles peuvent être également utilisées pour les cheveux sous forme de solutions aqueuses, alcooliques ou hydroalcooliques, ou sous forme de crèmes, de gels, d'émulsions, de mousses ou encore sous forme de compositions pour aérosol comprenant également un agent propulseur sous pression.

Pour l'injection, la composition peut se présenter sous forme de lotion aqueuse, de suspension huileuse ou sous forme de sérum. Pour les yeux, elle peut se présenter sous forme de gouttes et pour l'ingestion, elle peut se présenter sous forme de capsules, de granulés de sirops ou de comprimés.

Les quantités des différents constituants des compositions selon l'invention sont celles classiquement utilisées dans les domaines considérés.

Ces compositions constituent notamment des crèmes de nettoyage, de protection, de traitement ou de soin pour le visage, pour les mains, pour les pieds, pour les grands plis anatomiques ou pour le corps, (par exemple crèmes de jour, crèmes de nuit, crèmes démaquillantes, crèmes de fond de teint, crèmes anti-solaires), des fonds de teint fluides, des laits de démaquillage, des laits corporels de protection ou de soin, des laits anti-solaires, des lotions, gels ou mousses pour le soin de la peau, comme des lotions de nettoyage, des lotions anti-solaires, des lotions de bronzage artificiel, des compositions pour le bain, des compositions désodorisantes comprenant un agent bactéricide, des gels ou lotions après-rasage, des crèmes épilatoires, des compositions contre les piqûres d'insectes, des compositions anti-douleur, des compositions pour traiter certaines maladies de la peau comme l'eczéma, la rosasée, le psoriasis, les lichens, les prurits sévères.

'Les compositions peuvent également consister en des préparations solides constituant des savons ou des pains de nettoyage.

Les compositions peuvent aussi être conditionnées sous forme de composition pour aérosol comprenant également un agent propulseur sous pression.

L'inhibiteur de NO-synthase utilisé selon l'invention peut aussi être incorporé dans diverses compositions pour soins capillaires, et notamment des shampooings, des lotions de mise en plis, des lotions traitantes, des crèmes ou des gels coiffants, des compositions de teintures (notamment teintures d'oxydation) éventuellement sous forme de shampooings colorants, des lotions restructurantes pour les cheveux, des compositions de permanente (notamment des compositions pour le premier temps d'une permanente), des lotions ou des gels antichute, des shampooings antiparasitaires, etc.

Les compositions peuvent aussi être à usage bucco-dentaire, par exemple une pâte dentifrice. Dans ce cas, les compositions peuvent contenir des adjuvants et additifs usuels pour les compositions à usage buccal et notamment des agents tensioactifs, des agents épaississants, des agents humectants, des agents de polissage tels que la silice, divers ingrédients actifs comme les fluorures, en particulier le fluorure de sodium, et éventuellement des agents édulcorants comme le saccharinate de sodium.

Lorsque la composition est une émulsion, la proportion de la phase grasse peut aller de 5 % à 80 % en poids, et de préférence de 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.
Lorsque la composition est une solution ou un gel huileux, la phase grasse peut représenter plus de 90 % du poids total de la composition.

De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01 % à 10 % du poids total de la composition. Ces adjuvants, selon leur nature, peuvent être introduits dans la phase grasse, dans la phase aqueuse et/ou dans les sphérules lipidiques.

Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol, le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

La composition peut contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxyacides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

Selon l'invention on peut, entre autres, associer au moins un inhibiteur de NO-synthase à d'autres agents actifs destinés notamment à la prévention et/ou au traitement des affections cutanées.

Parmi ces agents actifs, on peut citer à titre d'exemple :
- les agents modulant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents antiviraux tels que l'acyclovir ;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés ;
- les agents antiprurigineux comme la thénaldine, la triméprazine ou la cyproheptadine ;
- les agents kératolytiques tels que les acides alpha- et bêta-hydroxycarboxyliques ou bêta-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'alpha-tocophérol ou ses esters, les superoxyde dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme l'acide rétinoïque ou le peroxyde de benzoyle.

Ainsi, selon un mode particulier, l'invention concerne l'utilisation d'au moins un inhibiteur de NO-synthase dans une composition comprenant au moins un agent choisi parmi les agents antibactériens, antiparasitaires, antifongiques, antiviraux anti-inflammatoires, antiprurigineux, anesthésiques, kératolytiques, anti-radicaux libres, anti-séborrhéiques, antipelliculaires, antiacnéiques et/ou les agents modulant la différenciation et/ou la prolifération et/ou la pigmentation cutanée.

Les exemples de compositions suivants illustrent l'invention sans la limiter aucunement. Les proportions indiquées sont des pourcentages en poids.
Ces compositions ont été obtenues par simple mélange des différents composants.

| Composition 1 : Lotion démaquillante pour le visage | |
|---|---|
| N^{G}-monométhyl-L-arginine (NMMA) | 10⁻⁵% |
| Antioxydant | 0,05 % |
| Isopropanol | 40,00 % |
| Conservateur | 0,30 % |
| Eau qsp | 100 % |

| Composition 2 : Gel pour le soin du visage | |
|---|---|
| 7-nitroindazole | 10⁻⁴ % |
| Hydroxypropylcellulose (Klucel H vendu par la société Hercules) | 1,00 % |
| Antioxydant | 0,05 % |
| Isopropanol | 40,00 % |
| Conservateur | 0,30 % |
| Eau qsp | 100 % |

| Composition 3 : Crème de soin du visage (émulsion huile dans eau) | |
|---|---|
| NG,NG-diméthyl-L-arginine | 10⁻² % |
| Stéarate de glycérol | 2,00 % |
| Polysorbate 60 (Tween 60 vendu par la société ICI) | 1,00 % |
| Acide stéarique | 1,40 % |
| Triéthanolamine | 0,70 % |
| Carbomer | 0,40 % |
| Fraction liquide du beurre de karité | 12,00 % |
| Perhydrosqualène | 12,00 % |
| Antioxydant | 0,05 % |
| Parfum | 0,50 % |
| Conservateur | 0,30 % |
| Eau qsp | 100 % |

| Composition 4 : Shampooing | |
|---|---|
| NG,NG-diméthyl-L-arginine | 10⁻⁵ % |
| Lauryl éther sulfate de Na (2,2 OE) | 12,00 % |
| Hydroxypropylcellulose (Klucel H vendu par la société Hercules) | 1,00 % |
| Parfum | 0,50 % |
| Conservateur | 0,30 % |
| Eau qsp | 100 % |

| Composition 5 : Gel anti-douleur | |
|---|---|
| N^{G}-monométhyl-L-arginine (NMMA) | 1,00 % |
| Hydroxypropylcellulose (Klucel H vendu par la société Hercules) | 1,00 % |
| Antioxydant | 0,05 % |
| Chlorhydrate de lidocaïne | 2,00 % |
| Isopropanol | 40,00 % |
| Conservateur | 0,30% |
| Eau qsp | 100 % |

| Composition 6 : Crème de soin de l'érythème solaire (émulsion huile-dans-eau) | |
|---|---|
| 7-nitroindazole | 0,50 % |
| Stéarate de glycérol | 2,00 % |
| Polysorbate 60 (Tween 60 vendu par la société ICI) | 1,00 % |
| Acide stéarique | 1,40 % |
| Acide glycyrrhétinique | 2,00 % |
| Triéthanolamine | 0,70 % |
| Carbomer | 0,40 % |
| Fraction liquide du beurre de karité | 12,00 % |
| Huile de tournesol | 10,00% |
| Antioxydant | 0,05 % |
| Parfum | 0,50 % |
| Conservateur | 0,30 % |
| Eau qsp | 100 % |

## Revendications

1. Utilisation d'au moins un inhibiteur de NO-synthase pour la préparation d'une composition cosmétique ou pharmaceutique destinée au traitement des peaux irritables et/ou réactives et/ou intolérantes.

2. Utilisation selon la revendication précédente, **caractérisée en ce que** la composition pharmaceutique est destinée à prévenir et/ou lutter contre les irritations cutanées et/ou les dartres et/ou les érythèmes et/ou les sensations dysesthésiques et/ou les sensations d'échauffement et/ou les prurits de la peau et/ou les muqueuses.

3. Utilisation selon l'une quelconque des revendications 1 ou 2, **caractérisée en ce que** l'inhibiteur de NO-synthase est utilisé en une quantité représentant de 10⁻⁶ % à 10 % du poids total de la composition.

4. Utilisation selon la revendication 3, **caractérisée en ce que** l'inhibiteur de NO-synthase est utilisé en une quantité représentant de 10⁻⁴ % à 1 % du poids total de la composition.

5. Utilisation selon l'une quelconque des revendications précédentes, **caractérisée en ce que** l'inhibiteur de NO-synthase est choisi parmi la N^{G}-monométhyl-L-arginine, l'ester méthyle de la N^{G}-nitro-L-arginine, la N^{G}-nitro-L-arginine, le chlorure de diphénylèneiodonium, la 7-nitroindazole, la N(5)-(1-iminoéthyl)-L-ornithine, le 2-(4-carboxyphényl)-4,4,5,5-tetraméthylimidazoline-1-oxy-3-oxyde, la NG,NG-diméthyl-L-arginine, la NG,NG-diméthyl-arginine, l'aminoguanidine, la canavanine et l'ebselen.

6. Utilisation selon la revendication 5, **caractérisée en ce que** l'inhibiteur de NO-synthase est l'ester méthyle de la N^{G}-nitro-L-arginine ou la NG,NG-diméthylarginine.

7. Utilisation selon l'une des revendications précédentes, **caractérisée en ce que** la composition est destinée à un usage topique.

## Patentansprüche

1. Verwendung mindestens eines Inhibitors der NO-Synthase zur Herstellung einer kosmetischen oder pharmazeutischen Zusammensetzung, die zur Behandlung von reizbarer und/oder reaktiver und/oder intoleranter Haut vorgesehen ist.

2. Verwendung nach dem vorhergehenden Anspruch, **dadurch gekennzeichnet, dass** die pharmazeutische Zusammensetzung zur Behandlung und/oder Vorbeugung von Hautirritationen und/oder Flechten und/oder Erythemen und/oder dysästhesischen Empfindungen und/oder Hitzeempfindungen und/oder Pruriti der Haut und/oder der Schleimhäute vorgesehen ist.

3. Verwendung nach einem der Ansprüche 1 oder 2, **dadurch gekennzeichnet, dass** der Inhibitor der NO-Synthase in einer Menge von 10⁻⁶ bis 10 % des Gesamtgewichts der Zusammensetzung verwendet wird.

4. Verwendung nach Anspruch 3, **dadurch gekennzeichnet, dass** der Inhibitor der NO-Synthase in einer Menge von 10⁻⁴ bis 1 % des Gesamtgewichts der Zusammensetzung verwendet wird.

5. Verwendung nach einem der vorhergehenden Ansprüche, **dadurch gekennzeichnet, dass** der Inhibitor der NO-Synthase unter N^{G}-Monomethyl-L-arginin, dem Methylester von N^{G}-Nitro-L-arginin, NG-Nitro-L-arginin, Diphenyleniodoniumchlorid, 7-Nitroindazol, N(5)-(1-Iminoethyl)-L-ornithin, 2-(4-Carboxyphenyl)-4,4,5,5-tetramethylimidazolin-1-oxy-3-oxid, N^{G},N^{G}-Dimethyl-L-arginin, N^{G},N^{G}-Dimethyl-arginin, Aminoguanidin, Canavanin und Ebselen ausgewählt ist.

6. Verwendung nach Anspruch 5, **dadurch gekennzeichnet, dass** es sich bei dem Inhibitor der NO-Synthase um den Methylester von N^{G}-Nitro-L-arginin oder N^{G},N^{G}-Dimethyl-arginin handelt.

7. Verwendung nach einem der vorhergehenden Ansprüche, **dadurch gekennzeichnet, dass** die Zusammensetzung zur topischen Anwendung vorgesehen ist.

## Claims

1. Use of at least one NO synthase inhibitor for the preparation of a cosmetic or pharmaceutical composition intended for the treatment of irritable and/or reactive and/or intolerant skins.

2. Use according to the preceding claim, **characterized in that** the pharmaceutical composition is intended to prevent and/or combat cutaneous irritations and/or scurf and/or erythema and/or dysaesthetic sensations and/or sensations of heating and/or pruritus of the skin and/or the mucous membranes.

3. Use according to either one of Claims 1 and 2, **characterized in that** the NO synthase inhibitor is used in a quantity representing from 10⁻⁶% to 10% of the total weight of the composition.

4. Use according to Claim 3, **characterized in that** the NO synthase inhibitor is used in a quantity representing from 10⁻⁴% to 1% of the total weight of the composition.

5. Use according to any one of the preceding claims, **characterized in that** the NO synthase inhibitor is chosen amongst N^{G}-monomethyl-L-arginine, N^{G}-nitro-L-arginine methyl ester, N^{G}-nitro-L-arginine, diphenyleneiodonium chloride, 7-nitroindazole, N(5)-(1-iminoethyl)-L-ornithine, 2-(4-carboxyphenyl)-4,4,5,5-tetramethylimidazoline-1-oxy 3-oxide, N^{G},N^{G}-dimethyl-L-arginine, N^{G},N^{G}-dimethylarginine, aminoguanidine, canavanine and ebselen.

6. Use according to Claim 5, **characterized in that** the NO synthase inhibitor is N^{G}-nitro-L-arginine methyl ester or N^{G},N^{G}-dimethylarginine.

7. Use according to one of the preceding claims, **characterized in that** the composition is intended for topical use.
